(19) Europäisches Patentamt
European Patent Office
Office européen des brevets

(11) **EP 4 647 014 A1**

(12) **EUROPEAN PATENT APPLICATION**
published in accordance with Art. 153(4) EPC

(43) Date of publication:
**12.11.2025 Bulletin 2025/46**

(21) Application number: **24756882.7**

(22) Date of filing: **13.02.2024**

(51) International Patent Classification (IPC):
***A61B 8/00*** *(2006.01)*

(52) Cooperative Patent Classification (CPC):
**A61B 8/00**

(86) International application number:
**PCT/JP2024/004878**

(87) International publication number:
**WO 2024/172039 (22.08.2024 Gazette 2024/34)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **17.02.2023   JP 2023023525**

(71) Applicant: **Gifts Inc.**
**Tokyo 162-0056 (JP)**

(72) Inventor: **OGASAWARA Jun**
**Tokyo 162-0056 (JP)**

(74) Representative: **Weickmann & Weickmann
PartmbB
Postfach 860 820
81635 München (DE)**

(54) **ULTRASOUND DIAGNOSTIC DEVICE, SHAPE ESTIMATION DEVICE, AND SYSTEM INCLUDING SAID DEVICES**

(57)     Provided is a method, device, and program that acquires multidirectional signals without requiring scanning, using multiple ultrasound probes embedded in a mat wrapped around body tissue for the construction and analysis of a three-dimensional structural model within the region of interest and transmission of results to users and shared recipients.

The multiple ultrasound probes (3) are embedded within a flexible mat (2) that conforms to the shape of the body tissue and adheres closely to its surface. The ultrasound probes (3) transmit and receive multiple ultrasound reflection images, which are then processed by a computing unit (4) to integrate the data into a 3D structural model of the region of interest.

The form, dynamics, and composition of the 3D structural model are automatically evaluated by the program, and the results are transmitted to the user's display or shared with remote systems.

FIG. 2

EP 4 647 014 A1

## Description

Technical Field

The present invention relates to an ultrasound diagnostic device that does not require scanning of an ultrasound probe. Furthermore, the invention relates to a method, device, and program for reconstructing the three-dimensional structure of body tissue using ultrasound irradiation from multiple directions and evaluating its morphology and dynamics.

Background

In conventional ultrasound examinations, a doctor or a technician scans an ultrasound probe over the surface of body tissue to capture images. Obtaining high-quality and comprehensive images requires advanced expertise and is subject to limitations in location and time. Additionally, ultrasound irradiation from a single direction has inherent limitations, such as artifacts caused by reflections from structures with high acoustic impedance and attenuation of ultrasound signals, leading to unclear images as the depth increases (Patent Documents 1-3).

Prior Art Documents

Patent Documents

Patent Document 1: Japanese Utility Model Publication No. S56-155808
Patent Document 2: Japanese Patent Application Publication No. 2005-137581
Patent Document 3: Japanese Patent Application Publication No. 2015-107311

Summary of the Invention

Problem to Be Solved by the Invention

In conventional ultrasound examinations (Patent Documents 1-3), a separate operator was required in addition to the examinee. Furthermore, examination results varied depending on the skill level of the operator, and information on the probe's position and scanning angle on the body tissue surface was lost in the obtained dynamic images, leading to issues in reproducibility and quantification.

Therefore, the objective of the present invention is to provide a device that enables an examinee to use the device independently by wrapping it around the target body tissue, where the obtained signals are automatically analyzed and evaluated, allowing for examinations without requiring specialized training.

Means for Solving the Problem

To achieve the above objective, the present invention is characterized by the following technical features.

In the present invention, ultrasound emitted from multiple ultrasound probes is received by multiple ultrasound probes, and a three-dimensional model is constructed by superimposing overlapping regions. This allows for structural evaluation within the region of interest without requiring automatic or manual scanning of the probe or oscillation of the ultrasonic transducers inside the probe.

Additionally, in the present invention, ultrasound emitted from a single probe is received multiple times from different locations, and a three-dimensional model is constructed by superimposing overlapping regions. The locations for multiple ultrasound transmissions and receptions are automatically calculated using machine learning or a predetermined method. Furthermore, the probe transmits and receives ultrasound only when it recognizes the optimal timing and position, allowing for structural evaluation within the region of interest without requiring manual scanning of the probe or oscillation of the ultrasonic transducers inside the probe.

At least one ultrasound probe (3) is embedded in a planar material (hereinafter referred to as a "mat") that has flexibility and adhesiveness for wrapping around body tissue. When multiple ultrasound probes (3) are present, they transmit and receive ultrasound at time intervals based on a specific frequency and phase to avoid interference. Ultrasound emitted from one probe and reflected within body tissue is received by both the transmitting probe and other probes at different positions. This information is integrated spatiotemporally by a computing device to create a three-dimensional structural model.

The mat's attachment direction and position are predetermined based on the classification of the examined body tissue (e.g., neck, abdomen, thigh). Since the relative positions of the probes change depending on the shape of the examined body tissue surface, the mat incorporates bending and extension sensors to calculate the mat's curvature and extension degree, thereby estimating the relative positions and angles of the probes. Based on this estimation, signals obtained from different probe pairs are superimposed to spatially integrate overlapping regions using an image-stitching approach.

By rapidly repeating multi-directional ultrasound transmission and reception and signal integration, dynamic changes in the three-dimensional structural model are depicted. A sufficiently high refresh rate enables the evaluation of fetal breathing-like movements and limb motions within the pregnant abdomen, facilitating fetal health assessments. Additionally, although conventional methods required ultrasound to be incident parallel to the blood flow to evaluate blood flow velocity using the Doppler effect, the present invention allows the estimation of hemodynamics from any cross-section and angle within the three-dimensional structural model constructed by integrating signals obtained from multiple directions.

The stiffness and composition of structures within body tissue can be estimated by measuring the propagation speed of shear waves generated by acoustic radiation pressure at angles different from the ultrasound propagation direction. In the present invention, since the transmitting and receiving ultrasound probes are located at different angles and positions relative to the region of interest, the stiffness and composition of body tissue, which were conventionally evaluated in a limited manner using unidirectional ultrasound probes, can be assessed more quantitatively and three-dimensionally.

If the density of ultrasound probes arranged on a plane is sufficiently high-specifically, comparable to the density of ultrasonic transducers in existing probes-comprehensive and highly precise reflection images within the region of interest can be obtained. Using this data as training data, the system can be trained to construct equivalent three-dimensional models even when signals are obtained from diagnostic devices with gradually reduced probe density. This enables a cost-effective arrangement while maintaining performance.

The invention allows users to independently wrap the mat around the target body tissue for examination. To prevent air from becoming trapped between the mat and the tissue surface, which could cause artifacts due to differences in acoustic impedance, the mat includes fine air vents and discharge grooves in areas not directly above the ultrasound probes. Additionally, an algorithm in the processing unit detects and excludes ultrasound probes affected by unresolved air bubbles from the integrated analysis.

The three-dimensional structural model and its morphological, dynamic, stiffness, and compositional information are transmitted to the user's display or remote information-sharing participants. Users can also opt to share data via external networks.

The invention consists of a planar mat with flexibility, skin adhesion, ultrasound transparency, and durability, multiple ultrasound probes with ultrasonic transducers, a processing unit, wired or wireless communication devices, wired or wireless power supply devices, and bending and extension sensors.

The main body is designed to be positioned and oriented according to the target body tissue site. The user attaches the main body around the body tissue, and air bubbles on the adhesion surface are naturally expelled through microscopic pores and grooves on the mat surface. Since the acoustic impedance of the mat is approximately equal to that of the human body, ultrasound emitted from the probe enters the body tissue with minimal artifacts.

Ultrasound propagating through the body tissue reflects at sites where acoustic impedance changes. The reflected waves are received by multiple probes, including the emitting probe. Based on ultrasound reflected at significantly different angles from the incident angle, acoustic radiation pressure and shear wave propagation speed are measured to estimate the hardness and composition of structures within the body tissue. The emission, reflection, reception, and attenuation of ultrasound from each probe are controlled with temporal and spatial offsets in the millisecond and millimeter ranges to prevent interference with subsequently emitted ultrasound. By transmitting and receiving ultrasound at a high refresh rate, the morphology and changes within the body tissue are captured.

By ensuring a sufficiently high refresh rate and an incident range that covers the region of interest, it is possible to capture the movement of body tissues that change over several seconds to tens of seconds, such as gallbladder contraction, gastrointestinal peristalsis, and uterine contractions.

Utilizing the Doppler effect and focused measurements on the region of interest enables the detection of hemodynamic changes, fetal heartbeats, and their instantaneous variations over shorter time frames.

Signals obtained from multiple ultrasound probes are transmitted either within the main body or to an external computing device via communication. These signals are integrated into a three-dimensional structural model using algorithms or pre-trained artificial intelligence.

The relative positions and angles of the ultrasound probes are estimated using flexion and extension sensors embedded in the mat. Furthermore, common regions in reflection images collected by adjacent ultrasound probes are overlaid, smoothed, and interpolated to construct a three-dimensional structural model of the region of interest.

The algorithm or artificial intelligence used to construct the three-dimensional structural model is designed to learn optimal parameters based on accumulated data, ensuring that performance does not degrade even with fewer input signals or computational resources. It is optimized with a balance of robustness against noise, energy efficiency, and accuracy.

The morphology, dynamics, hardness, and composition of the three-dimensional structural model are automatically evaluated and displayed on the user's local display, a remote computer used by an information-sharing party, or a cloud-based system. For example, fetal estimated weight may be calculated based on biparietal diameter, abdominal circumference, and femur length. Additionally, normality may be assessed based on respiratory-like movements, heart rate, limb and trunk movements, and amniotic fluid volume. These evaluation values are typically derived using conventional medical knowledge and calculation formulas. However, alternatively, machine learning may be employed to obtain evaluation values, using the generated three-dimensional structural model as explanatory variables and normality or estimated weight as objective variables.

If abnormal values are detected in the evaluation of the three-dimensional structural model, an alert indicating the presence of abnormal values is communicated to the user's local display, a remote computer used by an information-sharing party, or a cloud-based system.

Effects of the Invention

Since the system of this invention does not require an operator, examinations can be conducted regardless of the availability constraints of doctors and medical technicians, who are chronically in short supply. This reduces labor costs associated with highly paid medical professionals, leading to lower examination fees, which in turn facilitates the spread of screening tests and the early detection of diseases. Additionally, as the system operates without human intervention, it enables the collection and comparison of quantitative examination results. This allows for the aggregation of examination results across multiple facilities, supporting big data-based research. Furthermore, by reducing the labor required for examinations in labor-intensive medical environments, more time can be allocated to other tasks such as treatment and patient explanations. The system also has the advantages of reducing patient waiting times in outpatient clinics and hospital wards and mitigating regional disparities caused by variations in medical resource availability.

Brief Description of Drawings

FIG.1 is a block diagram illustrating an embodiment of the present invention.
FIG. 2 is an explanatory diagram showing a method of conducting an examination by wrapping the main body around the body tissue.
FIG. 3 is a diagram illustrating the arrangement of components such as the mat, multiple probes, flexion and extension sensors, processing unit, wired or wireless power supply, and wired or wireless communication device.
FIG. 4 is a flowchart depicting an ultrasound diagnostic method according to an embodiment of the present invention.
FIG. 5 is an explanatory diagram showing the integration of multiple signals within the main body or a cloud-based processing unit to construct a three-dimensional structural model of the region of interest.

Embodiments for Carrying Out the Invention

(Configuration of the Ultrasound Diagnostic System)

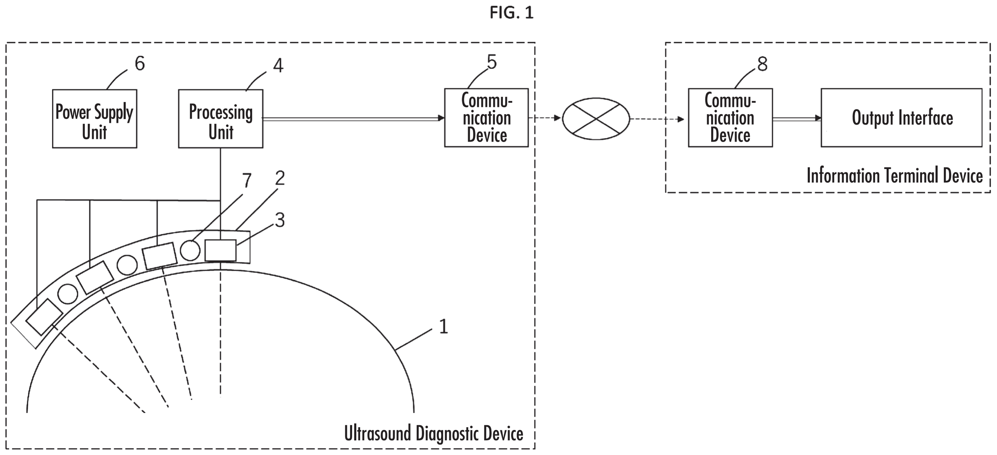

FIG. 1 presents a block diagram illustrating the overall configuration of the optimal ultrasound diagnostic system in the implementation of this invention. The main body (ultrasound diagnostic device) is installed without location restrictions, from medical institutions to patients' homes. In this embodiment, the region of interest is the upper abdomen, with the liver's three-dimensional structural model exemplified. However, this invention is not limited to liver imaging; it can also acquire three-dimensional structural models of the fetus, other organs, the thyroid gland, limb muscles, and other soft tissues. In such cases, the ultrasound diagnostic device described below is positioned not on the upper abdomen but on regions suitable for observing the fetus, organs other than the liver, the neck, or the limbs.

As shown in FIG. 1, the ultrasound diagnostic system of this embodiment comprises an ultrasound diagnostic device and an information terminal. The ultrasound diagnostic device includes a mat (2), ultrasound probes (3), flexion and extension sensors (7), a processing unit (4), a power supply unit (6), and a communication device (5). The signals transmitted from the ultrasound probes (3) or the three-dimensional structural models generated by the processing unit (4) (which may be a general-purpose computer or a cloud-based system) are evaluated in terms of morphology and dynamics by the processing unit (4). The results are then transmitted via wired or wireless communication to the communication device (8) of the information terminal, where they are displayed on the user's local display (output interface) or remotely for shared access.

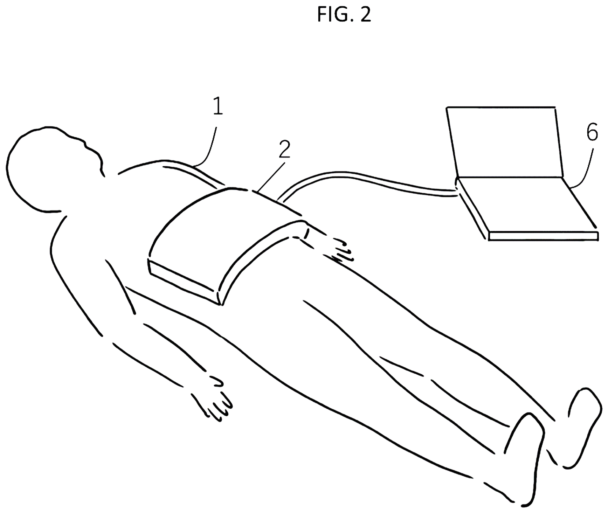

FIG. 2 illustrates a supine subject self-applying the mat (2) to the abdomen. The mat (2) is approximately 30-40 cm wide and 20-30 cm long. To avoid burdening the subject, the processing unit (4) or power supply unit (6) can be detached from the main body if weight is a concern. In FIG. 2, the power supply unit (6) and communication device (5) are integrated with the processing unit (4) and are connected via a wired connection to the ultrasound probes (3) embedded (preferably enclosed) within the mat (2). To prevent air bubbles from interfering with ultrasound transmission, the mat (2) has a fine structural surface that eliminates or naturally expels air bubbles at the contact interface with the body tissue (1). If unavoidable irregularities are present due to body hair, scars, or skin protrusions, a commercially available ultrasound gel with acoustic impedance similar to that of the human body may be applied.

Although the subject in FIG. 2 is in a supine position, the device can also be secured to the back or shoulders using a belt, allowing examinations in standing, sitting, or walking positions. This enables the evaluation of how gravity or the subject's body movements affect the organs or fetus within the region of interest.

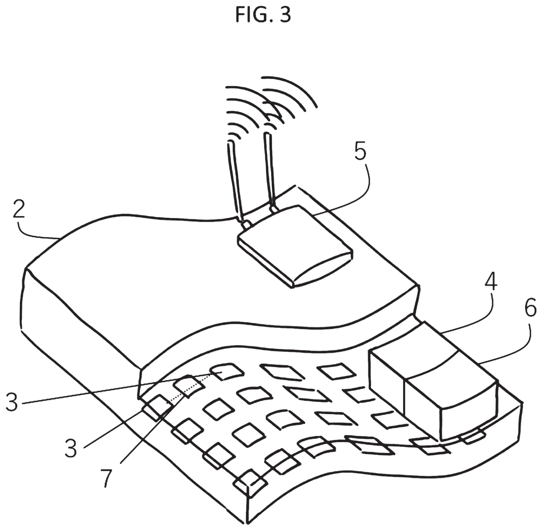

FIG. 3 presents an example of the arrangement and connections of the components that make up the main body. The mat (2) flexibly adapts to the shape of the target body tissue (1) and directs ultrasound into the region of interest. To estimate the relative positions and angles of the ultrasound probes (3), flexion and extension sensors (7) are embedded within the mat (2).

Regarding the placement of ultrasound probes (3), it is preferable to arrange them in a planar grid structure or a hexagonal close-packed structure to efficiently capture an approximately conical ultrasound field from a specific area of the human body. For example, the ultrasound probes (3) are preferably arranged in a lattice or honeycomb structure with mutual angles of approximately 60° or 90°, positioning each probe at the center points of the lattice or honeycomb structure.

Additionally, since the angle of the approximately conical spread is determined by the shape of the cone portion of the ultrasound probe (3), it is preferable for this angle to be configured between 30° and 120°, depending on the cone shape. Furthermore, to enhance measurement accuracy, each ultrasound probe (3) should ideally integrate at least two piezoelectric elements.

The optimal arrangement of multiple ultrasound probes (3) may be calculated by the ultrasound diagnostic device before they are placed. For instance, the placement of multiple ultrasound probes (3) may be estimated in such a way that maximizes the number of probes covering arbitrary curved surfaces within the target tissue. This allows for an arrangement that refines the resolution of the 3D model within the constraints of the maximum number of probes, thereby maximizing the resolution of the 3D model where possible.

The flexion and extension sensors (7) should ideally be placed at a layer distal to or at the same depth as the ultrasound probes (3) when viewed from the surface of the body tissue (1), connecting each ultrasound probe (3). For example, as shown in FIG. 3, when the ultrasound probes (3) are arranged in a lattice structure with approximately 90° angles, the flexion and extension sensors (7) should preferably be positioned at the midpoint of each honeycomb structure's center points. Alternatively, the flexion and extension sensors (7) may be laid out in a grid pattern independent of the ultrasound probe (3) arrangement. In this case, to minimize interference with the positions of the ultrasound probes (3), it is preferable to position the flexion and extension sensors (7) in a shallower layer than the ultrasound probes (3).

Furthermore, the arrangement and integration density of the ultrasound probes (3) vary depending on the purpose of the examination and the required accuracy. A high-density integration is used when a high spatial resolution is needed, while a low-density integration is used when the goal is to measure the gross movement or size of structures within the region of interest. This allows for an optimal balance between required performance and cost.

Additionally, the arrangement method or performance of the ultrasound probes (3) and flexion/extension sensors (7) may be determined through simulation within the intended scope.

The processing unit (4) and communication device (5) may be either built-in or removable depending on the purpose. For short-duration measurements or measurements taken during motion, the removal of heavier components enables weight reduction and energy efficiency. Similarly, power supply can be provided via a removable internal battery or through a wired connection.

(Ultrasound Diagnostic Method)

Next, the ultrasound diagnostic method implemented by the ultrasound diagnostic system of this invention is explained.

Each ultrasound probe (3) comprises an output unit (31) that generates ultrasound and an input unit (32) that captures ultrasound. The ultrasound generated by the output unit (31) is preferably a pulse wave, with an ideal frequency range of 1.5 MHz to 10 MHz, a pulse width of 16 to 512 nanoseconds, a pulse repetition period of 0.2 to 64 Hz, and a wave ripple length of 1 to 15.

Each ultrasound probe (3) may generate ultrasound waves of the same type. However, in the input unit, different probes (3) may generate different types of ultrasound to enable probe identification. Alternatively, even when generating the same type of ultrasound, each probe (3) may emit ultrasound at predetermined time intervals (e.g., 50 microseconds to 10 milliseconds, preferably 100 microseconds to 400 microseconds) to ensure they are distinguishable.

A portion of the ultrasound waves (input waves) emitted by the output unit (31) reaches the target tissue and forms reflection waves upon reflecting off the tissue surface. Another portion of the input waves scatters upon contacting the surface, forming scattered waves. Additionally, some of the input waves penetrate through the tissue as transmission waves without reflecting or scattering. Furthermore, input waves may refract due to density changes within the body tissue, forming refracted waves. The input unit (32) detects at least one of these wave types: reflection waves, scattered waves, transmission waves, or refracted waves. Simultaneously, the input unit (32) or the processing unit (4) identifies at least one of these waves.

The reflected wave is used to estimate the position and normal vector of a specific target point on the surface of the diagnostic target tissue. Specifically, when an input wave emitted from the output unit (31) of one ultrasound probe (3) is reflected at the target point, and the reflected wave is detected by another ultrasound probe (3) (or by the same probe if the input wave direction is parallel to the normal direction at the target point), the position vector t→ from the output unit (31) of the first ultrasound probe (3) to the target point on the diagnostic target tissue surface and the normal unit vector n→ at the target point can be estimated. Here, the position vector t→ satisfies the following equation (1), and the normal unit vector n→ satisfies equation (2):

$$p\rightarrow = t\rightarrow + r\rightarrow \qquad (Equation\ 1)$$

$$n\rightarrow = s\rightarrow + r\rightarrow \qquad (Equation\ 2)$$

Here, p represents the position vector from the output unit 31 of one ultrasound probe 3 to the input unit 32 of another ultrasound probe 3. The another ultrasound probe 3 in this context refers to the probe that detects the strongest reflected wave among multiple probes 3. r is the unit vector of the reflected wave that reaches the input unit 32 of this probe. s is the unit vector of the input wave emitted from the output unit 31 of the first ultrasound probe 3. Moreover, equation (1) can be rewritten as equation (3):

$$p = t + r = lt \times s + lr \times r \cdot \cdot \cdot (Equation\ 3)$$

Here, lt represents the magnitude of the position vector t (i.e., the distance from the first ultrasound probe 3 to the target point), and lr represents the magnitude of the position vector r (i.e., the distance from the other ultrasound probe 3 to the target point). Furthermore, since the following equation (4) is satisfied between the input wave unit vector s and the reflected wave unit vector r, the angle θ between s and the normal unit vector n can be determined:

$$s \cdot r = \cos(2\theta) \cdot \cdot \cdot (Equation\ 4)$$

(The " • " symbol denotes the dot product of the vectors.)

Additionally, the distances lt and lr satisfy the following equation (5):

$$lt + lr = c \times T \cdot \cdot \cdot (Equation\ 5)$$

Here, c is the speed of sound in the body, which typically ranges from 1400 to 1600 m/sec depending on frequency and tissue type. For certain tissues like bones, the speed of sound is calculated as 3500 to 4500 m/sec. T represents the time interval from when the input wave is emitted from the output unit 31 of the first ultrasound probe 3 until the reflected wave is detected by the input unit 32 of another ultrasound probe 3.

Using equations (1) to (5) described above, the position vector t and the normal unit vector n of the target point can be estimated from the known speed of sound c, time T, input wave unit vector s, reflected wave unit vector r, and position vector p.

Moreover, depending on the difference in acoustic impedance between an organ or a fetus and the surrounding body tissue, the reflected wave undergoes either fixed-end reflection or free-end reflection. By taking into account fixed-end or free-end reflection and incorporating variations in pulse wave phase, it is possible to distinguish between reflected waves and refracted waves, which will be discussed later.

The scattered wave is used to refine the estimation of the position and normal vector of a specific target point on the surface of the diagnostic target tissue under the assumption of Lambertian reflection. In a surface assumed to exhibit Lambertian reflection, a portion of the input wave diffuses uniformly in a hemispherical pattern due to Lambertian scattering. The reflectance of Lambertian reflection is known to satisfy the following equation (6):

$$i = \rho \times (n \cdot s) \cdot \cdot \cdot (Equation\ 6)$$

Here, i is the Lambertian diffuse reflectance, and $\rho$ is a proportional constant determined for each material. Since the Lambertian diffuse reflectance i depends on the input wave unit vector s and the normal unit vector n, the normal unit vector n can be estimated by ensuring that the Lambertian diffuse reflectance remains constant across multiple ultrasound probes 3 generating input wave unit vectors s with different orientations. Furthermore, the roughness of the target point can also be estimated from the Lambertian diffuse reflectance at that point.

The transmitted wave, along with the refracted wave, is used to estimate the acoustic impedance (density distribution) within the body tissue. The input wave generated at the output unit (31) of the ultrasound probe (3) may undergo refraction depending on the differences in acoustic impedance within the body tissue. Additionally, part of the input wave may propagate through the tissue without undergoing refraction, reflection, or scattering. The relative angle between one ultrasound probe (3) (which generates the input wave) and another ultrasound probe (3) (which receives the wave), represented as the angle $\theta$ between the input wave unit vector s and the normal unit vector n, as well as the distance (the magnitude of the position vector p), can be obtained using the previously described method. From this, information regarding whether the input wave refracts or travels straight can be obtained. The propagation time of the input wave between the ultrasound probes (3) is determined from real data. Using the distance between one ultrasound probe (3) and another ultrasound probe (3) along with the propagation time, the speed of sound along the ultrasound wave path is calculated. Since the speed of sound depends on the density of the medium through which it propagates, the average density along the path can be estimated. By calculating the average density along multiple ultrasound probe paths, the density distribution within the body tissue can be determined.

Next, the method for generating a three-dimensional (3D) model of the diagnostic target tissue and estimating the mass of the 3D model in the ultrasound diagnostic system of the present invention is explained.

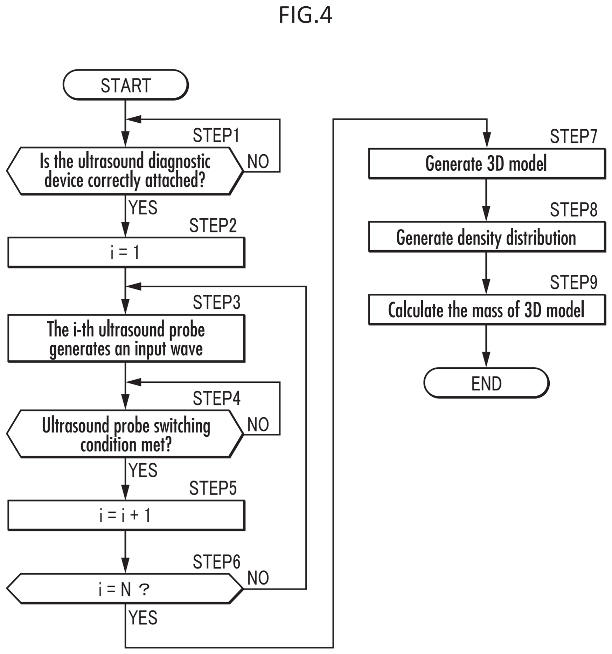

FIG. 4 is a flowchart illustrating the 3D model generation method and the mass estimation method of the generated 3D model in this invention. First, the diagnostic process of the ultrasound diagnostic device starts when the power is turned on (FIG. 4 / SSTART).

Next, it is determined whether the ultrasound diagnostic device is correctly attached (FIG. 4 / STEP 1). This determination may be performed automatically using signal-to-noise ratio analysis, or by evaluating the curvature of the ultrasound diagnostic device using multiple flexion and extension sensors (7). For example, the clinically relevant curvature of a pregnant abdomen typically falls within the range of R = 150 mm to 400 mm. Other automatic detection methods may include identifying significant deviations from a spherical surface, detecting local or reverse curvatures, using an unillustrated level sensor to check orientation, or determining cephalocaudal positioning based on the relative thickness between the subxiphoid region and the pubic symphysis. Additionally, STEP 1 may be performed manually or omitted entirely.

If the determination is negative (FIG. 4 / STEP 1: NO), the ultrasound diagnostic device returns to the previous step and executes STEP 1 again. At this point, an alert device may notify the user to adjust the device for proper positioning. Conversely, if the determination is positive (FIG. 4 / STEP 1: YES), the identifier i is set to 1 (FIG. 4 / STEP 2).

Next, a specific input wave is generated at the i-th ultrasound probe (3) among multiple ultrasound probes (FIG. 4 / STEP 3). As previously described, the input wave is preferably a specific pulse wave, and its frequency, amplitude, phase, and waveform may be adjusted depending on i. A combination of multiple sine waves may also be used to construct the pulse wave. When performing a Fourier transform on the pulse wave, the frequency and wavenumber may be set to depend on i. To ensure data reliability, the input wave may be generated multiple times. In this case, each generated input wave may be different or identical.

The arrangement of multiple ultrasound probes (3) and their identification numbers may be determined based on the probe layout. For example, in a grid layout, the ultrasound probe (3) located in the first row and first column may be assigned as the first ultrasound probe, while the probe located at row j and column k may be assigned as the ($\Sigma$ Jk-1 + k)-th ultrasound probe (Jk-1 represents the total number of columns up to k-1). Alternatively, the central ultrasound probe may be designated as the first ultrasound probe, with identification numbers assigned in a spiral pattern outward.

Additionally, the ultrasound probe (3) in the first row and first column may be designated as the first ultrasound probe, while the probe located farthest from it may be assigned as the second ultrasound probe. Similarly, the i-th ultrasound probe (3) may be the farthest probe at that step, while the i+1-th ultrasound probe is the next farthest probe that has not yet been assigned. In this case, since the input wave from the i-th ultrasound probe (3) weakens near the i+1-th ultrasound probe, noise is reduced, allowing the i+1-th ultrasound probe to generate its input wave more accurately. Furthermore, because the input wave from the i-th ultrasound probe (3) weakens near the i+1-th ultrasound probe, the ultrasound probe switching conditions described later are quickly met, thereby shortening the total ultrasound diagnostic time.

Moreover, the distance between the i-th and i+1-th ultrasound probes is denoted as $D_i$, and the sum ($\Sigma D_i$) (i = 1, N-1) may be maximized when assigning identification numbers up to i+1 = N. In this case, it is preferable to consider whether there are interference waves or multiple reflections that are more likely to persist due to the relative angle θ (the angle between the input wave unit vector s and the normal unit vector n) between the i-th and i+1-th ultrasound probes when assigning identification numbers. By doing so, input waves generated from the i-th ultrasound probe will minimize the effect of interference or multiple reflections, and the i+1-th ultrasound probe can generate its input wave more effectively. Additionally, controlling the emission sequence based on these identification numbers improves the time efficiency of the ultrasound diagnostic process.

In addition to the above method of assigning identification numbers, they may also be assigned randomly in space or concentrated in specific regions depending on the investigation target.

After the input wave is generated, it is received by multiple ultrasound probes (3) as a reflected wave, scattered wave, transmitted wave, or refracted wave. These waves are stored in an unillustrated memory unit as parameters for generating the 3D model or 3D density distribution, which will be described later. Subsequently, it is determined whether the ultrasound probe switching condition has been met (FIG. 4 / STEP 4).

Here, the "ultrasound probe switching condition" refers to the condition under which the ultrasound probe (3) generating the input wave switches from the i-th ultrasound probe (3) to the i+1-th ultrasound probe (3). For example, this condition may be met if a certain amount of time has passed since the i-th ultrasound probe (3) generated the input wave. Alternatively, the condition may be met when the receiving ultrasound probes, including the i-th probe, detect the arrival of an ultrasound signal.

Next, i+1 is assigned to the identifier i (FIG. 4 / STEP 5), and it is determined whether the current identifier i is equal to N (FIG. 4 / STEP 6). "N" preferably represents the total number of ultrasound probes (3) embedded in the ultrasound diagnostic device. However, it may also represent the minimum number of data acquisition cycles required to obtain sufficient ultrasound probe data for generating the 3D model or 3D density distribution. In other words, "N" does not necessarily have to be the total number of ultrasound probes (3) in the ultrasound diagnostic device.

If the determination is negative (FIG. 4 / STEP 6: NO), the process returns to just before STEP 3, and the procedures from STEP 3 onward are repeated. Conversely, if the determination is positive (FIG. 4 / STEP 6: YES), the process proceeds to STEP 7 and beyond.

In STEP 7, the ultrasound diagnostic device generates a 3D model of the diagnostic target tissue (FIG. 4 / STEP 7). Specifically, the information obtained in STEP 3 to STEP 4 is classified as at least one type among reflected waves, scattered waves, transmitted waves, and refracted waves. Based on at least one of these wave types, the system obtains data on the actual coordinate positions of multiple target points, normal unit vectors of multiple target points, roughness values of multiple target points, and density values of multiple target points. Using at least one of these data points, a 3D model of the diagnostic target tissue is generated.

Additionally, though optional, before or in parallel with STEP 7, a 3D density distribution of the diagnostic target tissue may be generated using at least one of the following data points: the actual coordinate positions of multiple target points, the normal unit vectors of multiple target points, the roughness values of multiple target points, and the density values of multiple target points (FIG. 4 / STEP 8).

Next, the mass of the 3D model is generated (FIG. 4 / STEP 9), and the ultrasound diagnostic method of this invention is completed (FIG. 4 / END). The mass of the 3D model may be calculated by integrating the density of each target point over infinitesimal volumes based on the 3D model and 3D density distribution. However, other methods may also be used. For example, mass may be calculated using mathematical formulas or statistical data based on arbitrary cross-sections of the 3D model. For instance, the volume, shape, or mass of the diagnostic target tissue may be estimated based on at least one of the following parameters from an arbitrary cross-section of the 3D model: major axis length, minor axis length, cross-sectional area, perimeter, curvature, and distance. In the case of a fetus, calculations may be performed using equation (7), which is the recommended formula by the Japan Society of Ultrasonics in Medicine, or by using the formula proposed by Shinozuka.

Estimated fetal weight (g) = 1.07 $\times$ (Biparietal diameter (cm))$^3$+0.30 $\times$ (Abdominal circumference (cm))$^2$ $\times$ (Femur length (cm))

That is, the mass may be calculated automatically by measuring the biparietal diameter, abdominal circumference, and femur length from the 3D model. Additionally, machine learning or deep learning may be used to set at least one of the following as explanatory variables: the actual coordinate positions of multiple target points, the normal unit vectors of multiple target points, the roughness values of multiple target points, and the density values of multiple target points. The target variables can include at least one of the following: the 3D model of the diagnostic target tissue, the 3D density distribution, or the mass of the diagnostic target tissue.

Although not illustrated, the processes from STEP 1 to STEP 9 may be repeated at a frequency of 0.2 to 64 times per second, preferably 2 to 16 times per second, to capture the temporal changes in the surface shape and mass of the 3D model of the diagnostic target tissue and estimate its dynamics. By comparing the morphology (volume, shape, and mass) and dynamics (temporal changes in volume, shape, and mass) of the diagnostic target tissue with clinical criteria or diagnostic standards, the system can determine normal or abnormal conditions and further assess the severity of abnormalities.

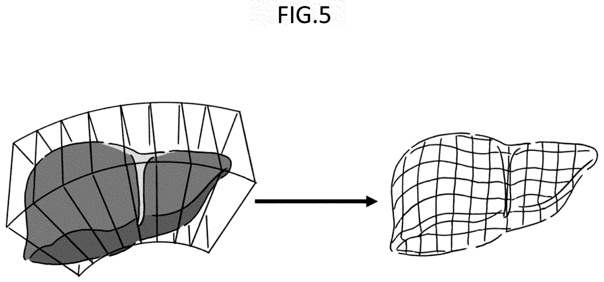

FIG. 5 illustrates the 3D structural model of the liver within the region of interest, as estimated using the aforementioned method, along with the collection of multi-directional ultrasound reflection images used for its construction. In this embodiment, the system evaluates the morphology of the liver, the course and hemodynamics of its vascular system (hepatic artery, hepatic vein, and portal vein), gallbladder contraction, liver tissue stiffness and fibrosis, fat deposition assessed through liver-kidney contrast, and liver mobility and adhesion due to respiration or body movement.

Each evaluation item is presented in general terms and indicators that non-medical users can understand. However, for medical professionals with whom the information is shared, more specialized terminology and indicators are provided.

Data from consenting examinees is stored in an internal memory or cloud storage and used to optimize the algorithm or artificial intelligence that constructs the 3D structural model.

The above description explains the automatic examination method, device, and program utilizing the ultrasound probes (3) of this invention based on specific embodiments. However, this invention is not limited to these embodiments and can be applied within the scope of the invention's objectives and technical domain. Additionally, the embodiments and usage examples of this invention may be modified or altered within a range that is easily adaptable by those skilled in the field.

Industrial Applicability

The automatic examination method, device, and program utilizing ultrasound probes (3) arranged on a flat surface in this invention do not require an operator other than the examinee. Therefore, examinations can be performed regardless of the availability of medical institutions or healthcare professionals, allowing rapid and automatic evaluation of body tissue. This offers various industrial applications, as described below.

The system can be used in medical underserved areas such as remote islands and rural areas, as well as during periods when supply cannot meet demand, such as during pandemics or disasters, even in urban areas. It is also useful in medical fields where the number of healthcare professionals is insufficient relative to patient demand, such as emergency medicine, obstetrics and gynecology, surgery, internal medicine, pediatrics, and home healthcare. The system aids in screening for diseases or disorders and determining severity, optimizing the allocation of human resources efficiently.

For examinees, unlike traditional outpatient or hospital examinations, they can complete the examination themselves. This reduces exposure to infectious diseases by minimizing contact opportunities and allows for quick testing without waiting time.

By providing various sizes of the device, examinations can be conducted for the neck, limbs, intra-abdominal organs, pelvic organs, pregnant abdomen and fetus, and fetal appendages. Evaluating the course of arteries and veins, stenosis, thrombi, and blood flow disturbances provides valuable information for preventing infarctions and planning rehabilitation. Assessing lower gastrointestinal motility and bowel content composition helps in preventing intestinal obstruction and improving bowel control. For thyroid examinations, the system enables rapid and labor-efficient evaluations of size, morphology, and composition.

For pregnant women, the system evaluates fetal respiratory-like movements, limb and trunk movements, heart rate, and amniotic fluid volume, enabling the estimation of fetal reserve. Additionally, by analyzing overall uterine dynamics to estimate contraction intensity, the system provides more detailed and real-time clinical information than traditional fetal heart rate monitoring (cardiotocography), widely used in pregnancy and labor management.

This system can detect pathological conditions such as fetal cerebral palsy or perinatal death, which can progress within minutes to tens of minutes, and issue alerts. Early detection leads to early intervention, improving perinatal outcomes.

As the system does not require an operator other than the examinee and adheres closely to body tissue, the examinee's posture is not limited to the usual supine position used in conventional examinations. Examinations can also be performed in standing, sitting, or walking postures.

The motion images, 3D structural models, and evaluation values obtained through this system provide quantitative data, unlike human-conducted examinations. This allows for research and education based on accumulated data.

The motion images, 3D structural models, and evaluation values obtained through this system can also be shared via social networking services (SNS) at the user's discretion.

By utilizing electromagnetic waves instead of ultrasound signals and applying this system to mechanical structures or buildings, it can be adapted for use in portable devices for inspecting the internal structure of machinery or buildings.

Explanation of Symbols

1 - Body tissue
2 - Mat
3 - Ultrasound probe
4 - Processing unit
5 - Communication device (ultrasound diagnostic device)
6 - Power supply unit
7 - Flexion and extension sensor
8 - Communication device (information terminal device)

**Claims**

1. An ultrasound diagnostic device, comprising:

   a flexible mat; and
   at least one ultrasound probe arranged in a planar
   configuration within the mat.

2. The ultrasound diagnostic device according to Claim 1, wherein,

   the mat is ultrasound-permeable, and
   the mat includes a discharge section that expels air bubbles generated at a contact surface with the body tissue
   surface.

3. The ultrasound diagnostic device according to Claim 1, wherein the mat includes a fixation section which is fixed to at least a portion of the body tissue surface.

4. The ultrasound diagnostic device according to Claim 1, wherein the ultrasound probe is installed within the mat.

5. The ultrasound diagnostic device according to Claim 1, wherein the ultrasound probe transmits and receives ultrasound waves at a specified frequency and phase.

6. The ultrasound diagnostic device according to Claim 1, wherein the ultrasound probe transmits and receives ultrasound waves at an optimal timing and position.

7. The ultrasound diagnostic device according to Claim 1, wherein, wherein an optimal arrangement of the ultrasound probes is computed.

8. The ultrasound diagnostic device according to Claim 1, further comprising a processing unit that constructs a three-dimensional structural model based on parameters obtained by the ultrasound probes.

9. The ultrasound diagnostic device according to Claim 1, further comprising a flexion sensor that identifies the position of the ultrasound probe.

10. A shape estimation device for estimating a shape of a three-dimensional structure, comprising:

an adhesion member that is attached to a target tissue surface; and
at least one signal transceiver installed in a planar configuration on the adhesion member.

11. The shape estimation device according to Claim 10, further comprising a processing unit that constructs a three-dimensional structural model based on parameters obtained by the signal transceiver.

12. The shape estimation device according to Claim 11, wherein the processing unit constructs the three-dimensional structural model using machine learning based on the obtained parameters.

13. The shape estimation device according to Claim 11, wherein the processing unit recognizes at least one of the form, dynamics, and composition of an entire or partial three-dimensional structural model.

14. A system comprising the shape estimation device according to any one of Claims 10 to 13, and

an information terminal device,
wherein the shape estimation device transmits the identified three-dimensional structural model to the information terminal device.

15. The system according to Claim 14, further comprising an anomaly detection device that detects abnormalities in the three-dimensional structural model,
wherein the detected abnormalities are transmitted to either the information terminal device or the shape estimation device.

FIG. 1

Power Supply Unit — 6

Processing Unit — 4

Communication Device — 5

Output Interface

Communication Device — 8

Information Terminal Device

7  2  3  1

Ultrasound Diagnostic Device

EP 4 647 014 A1

FIG. 2

FIG. 3

## FIG.4

START

STEP1
Is the ultrasound diagnostic device correctly attached? — NO

YES

STEP2
i = 1

STEP3
The i-th ultrasound probe generates an input wave

STEP4
Ultrasound probe switching condition met? — NO

YES

STEP5
i = i + 1

STEP6
i = N ? — NO

YES

STEP7
Generate 3D model

STEP8
Generate density distribution

STEP9
Calculate the mass of 3D model

END

FIG.5

## INTERNATIONAL SEARCH REPORT

| International application No. |
|---|
| **PCT/JP2024/004878** |

**A. CLASSIFICATION OF SUBJECT MATTER**

*A61B 8/00*(2006.01)i
FI: A61B8/00

According to International Patent Classification (IPC) or to both national classification and IPC

**B. FIELDS SEARCHED**

Minimum documentation searched (classification system followed by classification symbols)

A61B8/00-8/15

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | KR 10-2021-0110075 A (CHUNG ANG UNIVERSITY INDUSTRY ACADEMIC COOPERATION FOUNDATION) 07 September 2021 (2021-09-07) paragraphs [0038]-[0062], [0075]-[0078], [0081], fig. 2, 4 | 1, 4-8, 10-15 |
| Y | paragraphs [0038]-[0062], [0075]-[0078], [0081], fig. 2, 4 | 2-3, 9 |
| Y | JP 2018-519082 A (KONINKLIJKE PHILIPS N.V.) 19 July 2018 (2018-07-19) paragraphs [0020], [0023], [0027] | 2-3, 9 |
| Y | WO 2022/091174 A1 (ASAHI INTECC CO., LTD.) 05 May 2022 (2022-05-05) paragraph [0020] | 3 |
| A | JP 2008-538716 A (KONINKLIJKE PHILIPS ELECTRONICS N.V) 06 November 2008 (2008-11-06) entire text, all drawings | 1-15 |
| A | JP 2012-176197 A (NAGASAKI UNIVERSITY) 13 September 2012 (2012-09-13) entire text, all drawings | 1-15 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **17 April 2024** | **07 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

International application No.

**PCT/JP2024/004878**

**C.    DOCUMENTS CONSIDERED TO BE RELEVANT**

| Category* | Citation of document, with indication, where appropriate, of the relevant passages | Relevant to claim No. |
|---|---|---|
| A | JP 2016-528995 A (GASTER, Richard S.) 23 September 2016 (2016-09-23)<br>entire text, all drawings | 1-15 |
| A | JP 2012-239813 A (SONY CORPORATION) 10 December 2012 (2012-12-10)<br>entire text, all drawings | 1-15 |
| A | CN 109512461 A (ZHONGJU TECHNOLOGY CO., LTD.) 26 March 2019 (2019-03-26)<br>entire text, all drawings | 1-15 |

Form PCT/ISA/210 (second sheet) (July 2022)

**INTERNATIONAL SEARCH REPORT**

International application No.

**PCT/JP2024/004878**

| Box No. III | Observations where unity of invention is lacking (Continuation of item 3 of first sheet) |

This International Searching Authority found multiple inventions in this international application, as follows:

(Invention 1) Claims 1-9
　Claim 1 lacks novelty in light of document 1, and thus does not have a special technical feature. However, claim 2 dependent on claim 1 has the special technical feature in which the "mat is provided with a discharge portion for discharging bubbles occur on a surface in close contact with a biotissue surface."
　Therefore, claims 1-9 are classified as invention 1.

(Invention 2) Claims 10-15
　It cannot be said that claims 10-15 have the technical feature identical or corresponding to claims 1-9 classified as invention 1.
　Claims 10-15 are not substantially identical to or similarly closely related to any of the claims classified as invention 1.
　Therefore, claims 10-15 cannot be classified as invention 1.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

International application No.

**PCT/JP2024/004878**

| Patent document cited in search report | | | Publication date (day/month/year) | Patent family member(s) | | | Publication date (day/month/year) |
|---|---|---|---|---|---|---|---|
| KR 10-2021-0110075 | A | | 07 September 2021 | (Family: none) | | | |
| JP 2018-519082 | A | | 19 July 2018 | US 2018/0168544 paragraphs [0026], [0029], [0033] | A1 | | |
| | | | | WO 2017/001962 | A1 | | |
| | | | | EP 3316787 | A1 | | |
| | | | | CN 107809957 | A | | |
| WO 2022/091174 | A1 | | 05 May 2022 | US 2023/0255595 paragraph [0048] | A1 | | |
| | | | | EP 4233731 | A1 | | |
| | | | | CN 116419718 | A | | |
| JP 2008-538716 | A | | 06 November 2008 | US 2008/0304729 entire text, all drawings | A1 | | |
| | | | | WO 2006/114735 | A1 | | |
| | | | | EP 1890606 | A1 | | |
| | | | | KR 10-2008-0002857 | A | | |
| | | | | CN 101166473 | A | | |
| JP 2012-176197 | A | | 13 September 2012 | (Family: none) | | | |
| JP 2016-528995 | A | | 23 September 2016 | US 2016/0157717 entire text, all drawings | A1 | | |
| | | | | WO 2015/020886 | A1 | | |
| | | | | EP 3030147 | A4 | | |
| | | | | CA 2920566 | A1 | | |
| | | | | AU 2014304991 | A1 | | |
| | | | | KR 10-2016-0040593 | A | | |
| | | | | CN 105592786 | A | | |
| JP 2012-239813 | A | | 10 December 2012 | US 2012/0300578 entire text, all drawings | A1 | | |
| | | | | EP 2527830 | A2 | | |
| | | | | CN 102793562 | A | | |
| CN 109512461 | A | | 26 March 2019 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S56155808 U
- JP 2005137581 A

- JP 2015107311 A